# EUROPEAN PATENT SPECIFICATION

(11) **EP 1 804 852 B1**
(45) Date of publication and mention of the grant of the patent: **02.02.2011**
(21) Application number: 05798550.9
(22) Date of filing: 16.09.2005
(51) Int. Cl.: A61M 1/06, B65D 33/06, B65D 33/10, B65D 33/24, B65D 33/14

(54) **MILK BAG WITH PUMP ATTACHMENT STRAP**
MILCHBEUTEL MIT PUMPENBEFESTIGUNGSGURT
SAC A LAIT DOTE D'UNE BANDE DE FIXATION DE POMPE

(30) Priority: 21.09.2004 US 946251
(43) Date of publication of application: 11.07.2007
(73) Proprietor: Medela Holding AG, 6340 Baar (CH)
(72) Inventor: HUNT, Jill, M., Woodstock, IL 60098 (US)
(74) Representative: Clerc, Natalia
(86) International application number: PCT/US2005/033385
(87) International publication number: WO 2006/034138

(56) References cited:
- WO-A-94/26231
- WO-A-02/081003
- GB-A- 2 403 707
- US-A- 2 550 034
- US-A- 2 708 421
- US-A- 3 137 419
- US-A- 3 137 419
- US-A- 4 501 585
- US-A- 4 501 585
- US-A- 4 705 504
- US-A- 6 050 432
- US-B1- 6 576 278
- US-B1- 6 681 944

## Description

### FIELD OF THE INVENTION

The present invention generally relates to breastmilk pumps, and more particularly relates to an improved bag for attachment to a breastmilk pump. More specifically, the improved bag includes an attachment strap for securing the bag directly to a breastmilk pump, eliminating the need for a separate container for receiving expressed breastmilk.

### BACKGROUND OF THE INVENTION

Breastmilk pumps are well known and are generally comprised of a hood or shield that fits over a portion or the entire breast, a vacuum pump connected to the hood for generating an intermittent pressure variation within the hood, and a receptacle for the expressed milk. The receptacle in such an arrangement is typically a rigid plastic feeding bottle well known to those in the art. There are manually driven vacuum pumps (e.g., handheld piston pumps) which most commonly connect to at or closely adjacent to the hood, as well as vacuum pumps that are driven by an electric motor and interconnect to the hood via tubing. The vacuum pumps of these devices intermittently generate a pressure, most typically a vacuum (or a negative pressure) within the hood, with the hood encompassing the nipple and a substantial amount of the breast. The intermittent suction action of the pump serves to pull on the breast, drawing it within the narrowing funnel of the hood, to thereby extract milk in an action reminiscent of suckling. The milk so extracted typically flows from the hood into a container, e.g., a bottle, for storage and later use. A breastpump of the foregoing type is shown in U.S. Pat. No. 4,857,051. While rigid milk containers (bottles) are most often used with breastpumps, it is also desirable to use disposable plastic bags as the containers.

Many such sterile plastic bags have been proposed and adapted for use with a breast pump to function in the above-described manner. The method of using and storing these types of bags are well-documented as are the advantages. See for instance, U.S. Patent Publication No. 2002/0156419. However, without exception, these bags are used either independently of the pump, with milk from the container transferred to the plastic bag for storage, and/or used in combination with a container by, for example, having a portion adapted to be fixed in place by, for instance, a threaded connection with a collar or the like between the container and a housing or main portion of the pump. The threaded connection serves to fix the bag in place with respect to the pump, and the container provides support to the bag. The presence of the bag makes unnecessary the step of cleaning the container after use and, strictly speaking, makes the container merely a means of attaching the bag to the pump.

Applicants believe there is a demand for an improved disposable, sterile plastic bag with a feature that provides convenient use with a breast pump.

US 3,137,419 discloses a collapsible liquid container whith a retractable spout. The container comprises a handle reached on the closed part of the container. This container is used for liquid fuel as where it is necessary to convey a small quantity of fuel from a filling station to a motor vehicle.

US 2,708,421 discloses a dispensing device in the wise with the handle arranged on one side of the device.

US 6,681,944 discloses a breast-simulating nursing system with a strap member arranged on it.

WO 94/262 31 discloses a dispossible nursing bottle bag made of flexible material having a first end and a second end. The second end is sealed in a fluid tight fashion to form the bag bottom. The first end engages the open end of a holder. A pair of flaps is formed at the first end for assisting in opening the bag. The flaps are provided with a gripping feature for enhancing the gripping of the flaps.

WO 02/081003 dicloses a bag support for use with a breastmilk pump and a flexible bag.

### SUMMARY OF THE INVENTION

Now, with the foregoing in mind, the current invention builds upon the original concept of a disposable, sterile plastic bag for receiving and storing expressed breastmilk, and in the case of a preferred embodiment, there is provided a disposable, sterile bag including a pump attachment feature which does not require a container, collar or other element of the pump itself for attachment of the bag. It should be understood that the term "bag" as used herein is not intended to be limiting and is intended to refer to a receptacle adapted to use with a breast pump, and having a generally flexible structure and an interior to receive and retain breast milk during use and during storage. Of course, the term "bag" excludes conventional baby bottles and rigid containers.

One of the principal objects of the present invention is to provide a sanitary disposable bag for attachment to a breastmilk pump for containing breastmilk that can be easily and efficiently manufactured, packaged and used.

This object is achieved with a bag having the features of claim 1.

The inventive breastmilk bag comprises in one form an improved flexible plastic bag adapted to contain milk, such as a bag formed by two sheets of plastic constituting a front and a back sheet that are in facial engagement and are joined to each other by a series of seals in such manner to define a hermetically sealable liquid containing portion of the bag. The bag could also be formed by a continuous tube closed and separated in a well known manner, among other ways to form the general bag itself. One feature of the invention is a pump attachment element of the bag, which has a strap, sized and shaped to suspend the bag in place under a milk outlet of the pump. An embodiment of the invention includes a reclosable (preferably fluid tight when closed) opening for receiving expressed breastmilk when in an open condition, and sealing the bag when in a closed condition.

The attachment strap has first and second ends, the first end of which is attachable to one side (e.g., the front) of the bag and the second end is attachable to the other side (e.g., back) of the bag. The attachment strap is sized and shaped to position the bag to provide the opening in operative association with the breast pump, wherein the strap forms a hanger from which the bag is suspended in place on the pump.

An aspect of the present invention provides a bag for breastmilk in combination with a breastpump, including a breastpump with a housing having an outlet and a hood connected to the housing communicating expressed milk through to the outlet. The hood is sized and shaped to receive some or all of a breast. The bag has an opening sized and shaped to fit the outlet, and is otherwise closed. The attachment strap includes first and second ends. The first end can be fixed to the bag adjacent the opening in manufacture, for convenience of the user. It need not be, however, and could be provided as a separate piece. The attachment strap is sized and shaped to mount on the breastpump so as to suspend the bag in operative association with the breast pump and the opening fitted to the outlet. This can be done most preferably with a release paper covered adhesive on the second end, which when the paper is stripped way, reveals the adhesive for attachment to an opposed side of the bag. Other attachment schemes are readily adaptable, however. An aspect of the present invention is provided wherein the attachment strap is formed as a unitary part of the bag itself, i.e., integral with the bag material and not detachable from the bag.

The present invention will be further appreciated, and its attributes and advantages further understood, upon consideration of the following detailed description of an embodiment of the invention, taken in conjunction with the accompanying drawings, in which:

### BRIEF DESCRIPTION OF THE DRAWINGS

FIG. 1 is a front view of an embodiment of a breastmilk bag according to certain aspects of the present invention;
FIG. 1A is a sectional view taken along line A-A of FIG. 1;
FIG. 1B is a front view of another embodiment of a breastmilk bag according to certain aspects of the present invention;
FIG. 2 is a perspective view of an embodiment of the breastmilk bag prior to being mounted to a breastpump; and
FIG. 3 is a side view of an embodiment of the breastmilk bag of FIG. 1 mounted to the breastpump.

### DETAILED DESCRIPTION OF EMBODIMENTS OF THE INVENTION

The embodiments of the invention described hereinafter have been particularly adapted for use with a conventional, commercially available breastpump; it need not be conventional, however, so long as there is something from which the bag can be suspended by the strap in place around the milk outlet. The breastpump may be manual or motor driven.

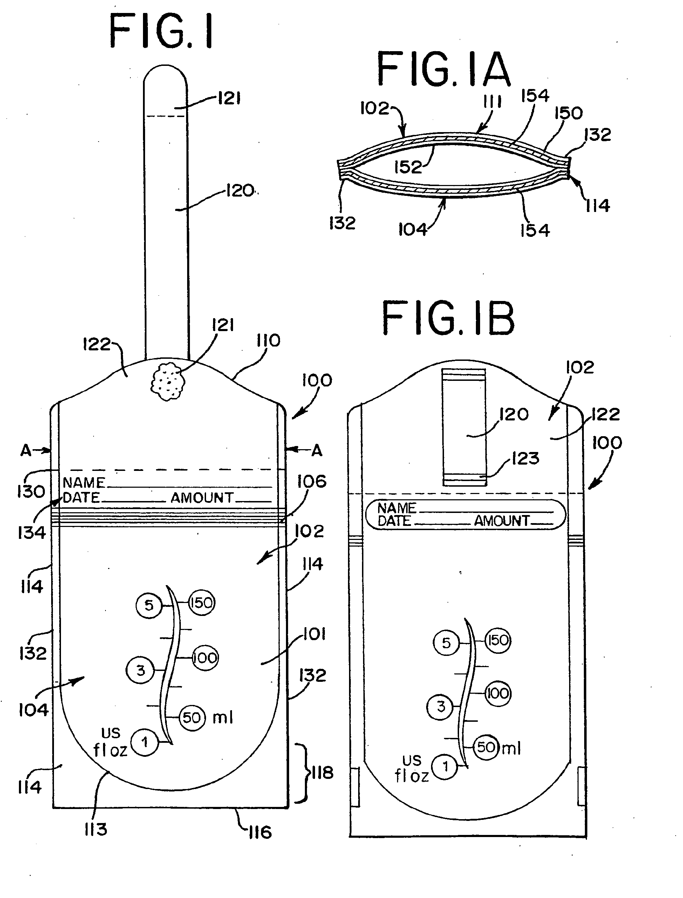

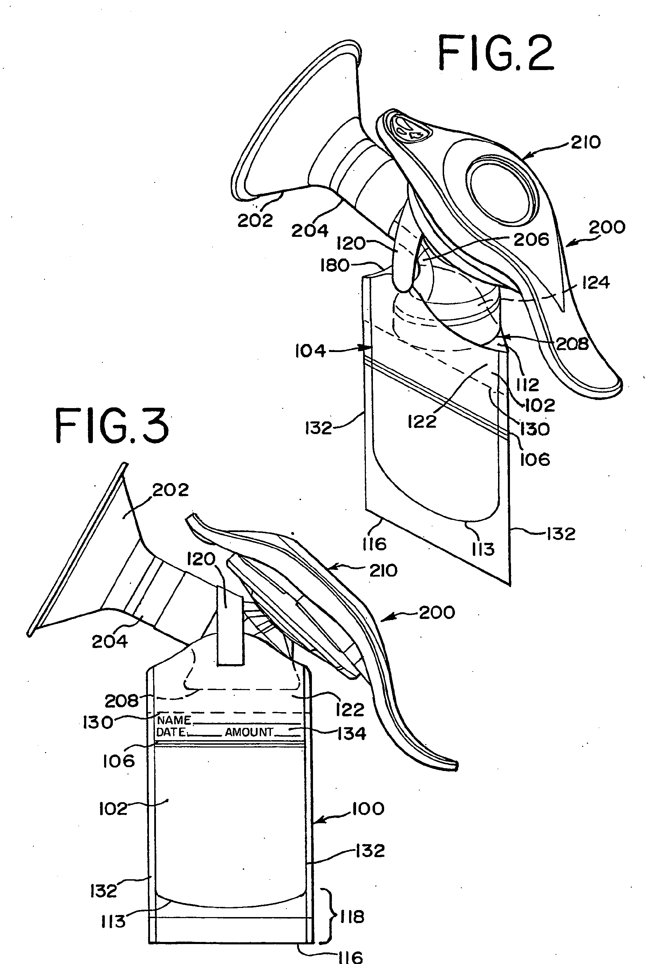

Referring to FIG. 1, a bag 100 for containing breastmilk comprises a front sheet 102 and a back sheet 104 (See FIG. 2). The front and back sheets 102, 104 are made of a suitable liquid impervious food compatible plastic, such as polyethylene. Each of sheet 102, 104 may be made from a single ply of material or, in the alternate a suitable laminate. A polyethylene-polyester laminate can be advantageously used, with the polyethylene layer on the inside of the bag for flexibility, and also better sealability. Each of front sheet 102 and rear sheet 104 (See FIG. 2) may be formed of a laminate including 44 gauge polyester (PET) film and 200 gauge low-density polyethylene (LDPE) film.

Looking at FIG. 1A in particular, one notes that each sheet 102, 104 is generally rectangular with a pair of opposed parallel side edges 132 and a bottom edge 116 (FIG. 1) set at right angles to side edges 132 and a top edge 110, which is slightly scalloped around a center area of the sheets at the opening. Each of the front sheet 102 and rear sheet 104 is formed of a laminate 111 comprising a first side 150 of a 44 gauge polyester (PET) film and a second side 152 including a 200 gauge low-density polyethylene (LDPE) film, first side 150 and second 152 joined by a tie layer 154. The densities of the film, and even the type of film (or other material), is not limiting, and is merely descriptive of this one embodiment.

Front sheet 102 and rear sheet 104 are joined by seal 114, which is applied to side edges 132 and (referring back to FIG. 1) bottom edge 116 forming a pouch 112 (FIG. 2) with opening 180 defined at top edge 110. A gusset structure 118 is formed between bottom edge 116 and bottom of pouch 102, formed by seal line 113. This gusseted bottom is formed by conventional methods, e.g., providing a folded panel of pouch material adjacent the bottom edge 116 of the bag and sealing the material into a gusset structure. It lends further strength to the region, and even the ability to stand the bag when filled. Suitable materials for making such disposable milk bags are well known. The bag 100 can similarly be formed from a continuous tube of plastic, eliminating the need for lateral seals for the bag.

A resealable or reclosable seal 106 is provided to provide access to the bag interior (pouch 112) when in an open condition and seals the bag when in a closed condition.

In one embodiment, the reclosable seal is preferably a "zip" type closure 106 or an equivalent, formed by attaching to either front sheet 102 or rear sheet 104, an extended male element, above and essentially parallel to bottom edge 116, which male element is press fit into a corresponding female channel type element attached to the other of the front and back sheet 102, 104. A number of known designs exist for "male/female" zip-type closures. Some are designed to fasten together to merely hold a bag opening in a closed condition and some are designed to retain fluids within the bag in a leak proof fashion. Of course, other mechanisms of sealing the bag are contemplated for use in the present invention to seal the bag in a reclosable fashion.

Reclosable seal 106 extends between side edges 132 and is also located below top edge 110. Thereby, when reclosable seal 106 is closed, the combination of reclosable seal 106, sealed side edges 132 and sealed pouch bottom edge 113 define the generally rectangular bag pouch 112 suitable for retaining fluids therewithin through opening 180.

Because reclosable seal 106 is positioned below top edge 110, a portion of each of front sheet 102 and rear sheet 104 extends beyond reclosable seal 106. The extended portions of sheets 102 and 104 form two opposing tabs or flaps, respectively; front tab 122 and rear tab 124. Front tab 122 and rear tab 124 are used to aid in opening bag 100, but as will now be discussed, also in positioning the bag in use.

Bag 100 includes a strap 120 preferably attached to front sheet 102 (See FIG. 2) by adhesive 121 at one end thereof, by heat sealing or any other suitable method or material adjacent upper part of tab 122. The strap 120 may be attached in a releasable fashion as well, e.g., looped through a hole(s). The other end of the strap 120 may include adhesive 121, or an equivalent attachment mechanism or material for releasable or permanent attachment to the rear sheet 104 on tab 124 adjacent the upper end thereof. The strap 120 may be a single layer of material, such as plastic or paper configured as a rectangular strap, may be multiple plies of material, may be string-like or any suitable shape so as to provide an attachment to a breastpump as will be discussed in more detail below.

Referring now in particular to FIG. 1B, a breastmilk receptacle or bag 100 is shown, which is similar to that shown in FIG. 1. The bag 100 illustrates one preferred embodiment of providing the strap 120 in a stored or pre-deployed condition. In particular, the strap 100 may be coiled or folded into a compacted form on the outside of front tab 122 of front sheet 102. The strap 120 is held in the folded condition by a temporary adhesive (not shown) or a layer of material 123 overlaying the strap, which is removed before use of the strap.

Referring now in particular to FIGS. 2 and 3, a breastpump 200 includes a funnel-shaped hood 202 that is sized and shaped for engagement with at least a portion of a breast. Downstream from the hood 202 is a cylindrical extension 204 which is connected or connectable to a receiving portion of a pump housing 206. Upstream and downstream are used relative to milk flow in use of the invention. The pump housing 206 includes a conduit (not shown) for conveying expressed breastmilk from the hood 202, through extension 204 and out through an outlet 208. In the given example, this is a lever pump mechanism 210 connected to the housing 206 to provide a cyclical negative pressure at the hood 202. Further details of this pump mechanism 210 and associated components, including valving, as well as operation, can be gleaned from U.S. Patent Application Publication No. 2004/0039330. Again, the specific breastpump is incidental to the inventive bag.

In use, longitudinal strap 120 is directed over the extension 204 and housing 206 so as to suspend the bag 100 below the pump 200 with the outlet 208 inserted into the bag interior (pouch 112). It will be understood that the opening to the bag interior formed in part by the tabs 122, 124, is sized and shaped so as to fit with the outlet 208 of pump housing 206 such that milk conveyed through the outlet is directed into the bag interior without spillage. The present invention contemplates a variety of cooperating configurations of the outlet 208 and bag 100. While the strap 120 is depicted as encircling the extension 204 and housing 206 of pump 200, the strap may engage the pump in other ways, which are also within the scope of the invention.

Strap 120 uses an adhesive covered by a release paper or sheet, which is commonly known. The release paper is removed to expose the adhesive 121 and then to affix the free end of the strap 120 to the tab 124. The strap 120 need not be provided already affixed at one end to the bag, but could be supplied separately, although attaching one end to the bag is considered more advantageous.

Strap 120 can be formed from several types of material. No one material or form is critical to the present invention. Neither the means of affixing the strap 120 to the tabs 122, 124 nor the shape or type of adhesive are critical to the present invention so long as the basic object of the invention is satisfied.

Returning to FIG. 1, for instance, each of front sheet 102 and rear sheet 104 includes a line of perforation 130, located upstream of and parallel to reclosable seal 106 and extending between side edges 132. By tearing along line of perforation 130, after filling and sealing bag 100, those parts of bag 100 which are not required for storage (i.e., tabs 122, 124), may be detached and discarded.

Bag 100 may further include a label area 134, located on the outer side of front sheet 102, of sufficient dimensions to allow for some information to be provided on bag 100 regarding the contents in the bag, such as, for example, "DATE", "VOLUME" and "NAME", either by writing directly on front sheet 102, or indirectly by adhering an optional label at this location. Other locations for information may be provided. Calibrated markings 101 as for milk volume may additionally be provided.

The present invention has been described and illustrated with reference to specific embodiments; those skilled in the art will recognize that modifications and variations may be made.

## Claims

1. A bag (100) for use with a breastpump, wherein the breastpump has an outlet through which milk travels and structure above the outlet, the bag (100) being
formed of material having an interior defined within the bag (100) to form a liquid containing portion (112), and defining an opening (180) to the bag (100), the bag further comprising an elongated attachment element, the elongated attachment element being an attachment strap (120), the attachment strap (120) being sized and shaped to be supported by the breastpump structure and position the bag under the outlet to receive milk therefrom, wherein the attachment strap includes first and second ends and wherein the strap forms a hanger from which the bag is suspended in place on the breastpump, **characterized in that** the first end being attachable or attached to one side (104) of the bag and the second end being attachable or attached to the other side (102) of the bag so as to form a loop over the bag opening (180).

2. The bag of Claim 1, further including a closable seal (106) positioned adjacent the opening (180).

3. The bag of Claim 2, wherein the closable seal (106) is a reclosable seal.

4. The bag of Claim 2, wherein the bag (100) includes a tear line (130) upstream from the closable seal (106).

5. The bag of Claim 4, wherein the attachment strap (120) has a first end permanently attached to said bag and a free end being joined to the bag in use to form the loop.

6. The bag of Claim 5, wherein the free end has an adhesive portion (121).

7. The bag of Claim 6, wherein the strap (120) is attached in use to a removable part of the bag that is upstream from the tear line (130), whereby the strap (120) and removable part are dissociable from the remainder of the bag along the tear line (130).

8. The bag of Claim 7, wherein the tear line (130) is a perforation positioned between the closable seal (106) and the opening (180).

9. The bag of Claim 1, wherein the second end is releasably attachable to the other side (102), wherein the second end is attachable to a rear sheet (104) on a tab (124) adjacent the upper end thereof.

## Patentansprüche

1. Beutel (100) zur Verwendung mit einer Brustpumpe, wobei die Brustpumpe einen Ausgang, durch welchen Milch läuft, und eine Struktur oberhalb des Ausgangs aufweist, wobei der Beutel (100)
aus Material hergestellt ist, dessen Innenseite im Beutel (100) definiert ist, um einen Flüssigkeit aufnehmenden Bereichs (112) zu bilden, und welches eine Öffnung (180) des Beutels (100) festlegt, wobei der Beutel weiters ein langgestrecktes Befestigungselement aufweist, bei welchem es sich um ein Befestigungsband (120) handelt, das bemessen und geformt ist, um von der Struktur der Brustpumpe getragen zu werden, und um den Beutel zur Aufnahme von Milch unter dem Ausgang zu positionieren, wobei das Befestigungsband erste und zweite Enden aufweist, und wobei das Band einen Anhänger bildet, von welchem der Beutel an einer Stelle der Brustpumpe herabhängt, **dadurch gekennzeichnet, dass** das erste Ende an einer Seite (104) des Beutels anbringbar oder angebracht ist und das zweite Ende an der anderen Seite (102) des Beutels anbringbar oder angebracht ist, so dass eine Schleife über der Beutelöffnung (180) geformt wird.

2. Beutel gemäss Anspruch 1, wobei der Beutel ausserdem eine verschliessbare Abdichtung (106) aufweist, die angrenzend an die Öffnung (180) angeordnet ist.

3. Beutel gemäss Anspruch 2, wobei die verschliessbare Abdichtung (106) wiederverschliessbar ist.

4. Beutel gemäss Anspruch 2, wobei der Beutel (100) eine Reisslinie (130) aufweist, welche stromaufwärts von der verschliessbaren Abdichtung (106) angeordnet ist.

5. Beutel gemäss Anspruch 4, wobei das Befestigungsband (120) ein erstes Ende hat, das dauerhaft an diesem Beutel angebracht ist, und ein freies Ende hat, das bei Gebrauch mit dem Beutel verbunden ist, um die Schleife zu formen.

6. Beutel gemäss Anspruch 5, wobei das freie Ende einen haftenden Bereich (121) aufweist.

7. Beutel gemäss Anspruch 6, wobei das Band (120) bei Gebrauch an einem entfernbaren Teil des Beutels angebracht ist, welcher stromaufwärts von der Reisslinie (130) angeordnet ist, und wobei das Band (120) und der entfernbare Teil vom Überbleibsel des Beutels entlang der Reisslinie (130) trennbar sind.

8. Beutel gemäss Anspruch 7, wobei die Reisslinie (130) eine Perforation ist, welche zwischen der verschliessbaren Abdichtung (106) und der Öffnung (180) angeordnet ist.

9. Beutel gemäss Anspruch 1, wobei das zweite Ende lösbar an der anderen Seite (102) anbringbar ist, und wobei das zweite Ende an eine hintere Folie (104) angrenzend an deren oberes Ende an einer Lasche (124) anbringbar ist.

## Revendications

1. Sac (100) destiné à l'utilisation avec un tire-lait, dans lequel le tire-lait présente une sortie à travers laquelle passe le lait et une structure au-dessus de la sortie, le sac (100) étant formé d'un matériau dont l'intérieur est défini dans le sac (100) de manière à former une portion contenant du liquide (112), et définissant une ouverture (180) du sac (100), le sac comprenant en outre un élément de fixation allongé, l'élément de fixation allongé étant une bande de fixation (120), la bande de fixation (120) étant dimensionnée et formée de manière à être supportée par la structure du tire-lait et de manière à positionner le sac sous la sortie afin d'en recevoir le lait, la bande de fixation comportant des première et deuxième extrémités et la bande formant un dispositif d'accrochage depuis lequel le sac est suspendu en position sur le tire-lait, **caractérisé en ce que** la première extrémité peut être attachée ou est attachée à un côté (104) du sac et la deuxième extrémité peut être attachée ou est attachée à l'autre côté (102) du sac de manière à former une boucle par-dessus l'ouverture du sac (180).

2. Sac selon la revendication 1, comportant en outre un joint fermable (106) positionné à côté de l'ouverture (180).

3. Sac selon la revendication 2, dans lequel le joint fermable (106) est un joint refermable.

4. Sac selon la revendication 2, dans lequel le sac (100) comporte une ligne de déchirure (130) en amont du joint fermable (106).

5. Sac selon la revendication 4, dans lequel la bande de fixation (120) présente une première extrémité attachée de manière permanente audit sac et une extrémité libre étant reliée au sac pendant l'utilisation de manière à former la boucle.

6. Sac selon la revendication 5, dans lequel l'extrémité libre a une portion adhésive (121).

7. Sac selon la revendication 6, dans lequel la bande (120) est attachée pendant l'utilisation à une partie amovible du sac qui est disposée en amont de la ligne de déchirure (130), la bande (120) et la partie amovible pouvant être séparées du reste du sac le long de la ligne de déchirure (130).

8. Sac selon la revendication 7, dans lequel la ligne de déchirure (130) est une perforation située entre le joint fermable (106) et l'ouverture (180).

9. Sac selon la revendication 1, dans lequel la deuxième extrémité peut être attachée de manière amovible à l'autre côté (102), la deuxième extrémité pouvant être attachée à une feuille arrière (104) sur une languette (124) à côté de l'extrémité supérieure de celle-ci.
